(19) Europäisches Patentamt
European Patent Office
Office européen des brevets

(11) Veröffentlichungsnummer: **0 287 988 A2**

# (12) EUROPÄISCHE PATENTANMELDUNG

(21) Anmeldenummer: **88106137.8**

(22) Anmeldetag: **18.04.88**

(51) Int. Cl.⁴: **C07D 207/333 , A61K 31/40**

(30) Priorität: **24.04.87 DE 3713725**

(43) Veröffentlichungstag der Anmeldung:
**26.10.88 Patentblatt 88/43**

(84) Benannte Vertragsstaaten:
**AT BE CH DE ES FR GB GR IT LI NL SE**

(71) Anmelder: **CASSELLA Aktiengesellschaft**
**Hanauer Landstrasse 526**
**D-6000 Frankfurt am Main 61(DE)**

(72) Erfinder: **Zoller, Gerhard, Dr.**
**Höhenstrasse 8**
**D-6369 Schöneck(DE)**
Erfinder: **Beyerle, Rudi, Dr.**
**An der Pfaffenmauer 44**
**D-6000 Frankfurt/Main 60(DE)**
Erfinder: **Schindler, Ursula, Dr.**
**Reinhardswaldweg 1**
**D-6082 Mörfelden-Walldorf(DE)**

(74) Vertreter: **Urbach, Hans-Georg, Dr. et al**
**Hanauer Landstrasse 526**
**D-6000 Frankfurt am Main 61(DE)**

## (54) Pyrrolaldehyde, ihre Herstellung und ihre Verwendung.

(57) Pyrrolaldehyde der allgemeinen Formel I

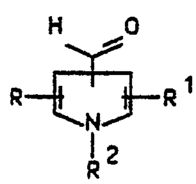

(I)

worin R und $R^1$ unabhängig voneinander Wasserstoff oder Alkyl mit 1 bis 4 C-Atomen,
$R^2$ Alkyl, das durch $-NH_2$, Acylamino der allgemeinen Formel II

$$-NH-\overset{X}{\overset{\|}{C}}-R^3 \quad (II)$$

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und
$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Phenylaminogruppe bedeutet, und deren pharmazeutisch annehmbare Säureadditionssalze, ihre Herstellung sowie ihre Verwendung.

EP 0 287 988 A2

## Pyrrolaldehyde, ihre Herstellung und ihre Verwendung

Die vorliegende Erfindung betrifft Pyrrolaldehyde der allgemeinen Formel I

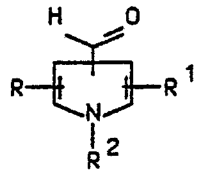 (I)

worin R und $R^1$ unabhängig voneinander Wasserstoff oder Alkyl mit 1 bis 4 C-Atomen,
$R^2$ Alkyl, das durch $-NH_2$, Acylamino der allgemeinen Formel II

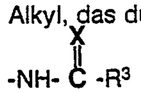 (II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und
$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Phenylaminogruppe bedeutet, sowie deren pharmazeutisch akzeptable Säureadditionssalze, wobei für die Fälle, in denen für $R^2$ stehendes Alkyl durch eine Carbonsäuregruppierung substituiert ist, für R und $R^1$ Alkyl stehen muß und wobei $R^3$ nicht für Wasserstoff stehen kann, wenn R und $R^1$ Wasserstoff bedeuten und der Pyrrolring in 3-Stellung formyliert ist. Die Erfindung betrifft auch Verfahren zur Herstellung der Verbindung der allgemeinen Formel I und ihrer Säureadditionssalze sowie ihre Verwendung als pharmakologische Wirkstoffe.

Insbesondere betrifft die Erfindung Pyrrolaldehyde der allgemeinen Formel I

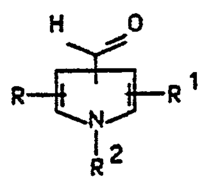 (I)

worin R und $R^1$ unabhängig voneinander (Wasserstoff oder Alkyl mit 1 bis 4 C-Atomen,
$R^2$ Alkyl mit 1 bis 3 C-Atomen, das durch $-NH_2$, Acylamino der allgemeinen Formel II

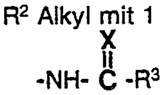 (II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeutet,
X für ein Sauerstoff-oder Schwefelatom steht und
$R^3$ Wasserstoff, Alkyl oder Alkylamino mit 1 bis 5, vorzugsweise 1 oder 2, C-Atomen, das gegebenenfalls durch $-NH_2$, Monoalkylamino mit 1 bis 4, vorzugsweise 1 oder 2, C-Atomen, Dialkylamino mit insgesamt 2 bis 6, vorzugsweise 2 bis 4, C-Atomen, einen N-Pyrrolidinyl-, N-Piperidinyl-, N-Morpholinyl-, N-Thiomorpholinyl-oder durch einen in 4-Stellung gegebenenfalls durch Alkyl mit 1 bis 4 C-Atomen, Phenyl, Toluyl, Chlorphenyl oder Methoxy-oder Ethoxyphenyl substituierten Piperazin-1-yl-Rest, durch Alkoxy mit 1 bis 4, vorzugsweise 1 oder 2, C-Atomen oder durch gegebenenfalls substituiertes Phenoxy substituiert ist; Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen. eine Aminogruppe oder eine gegebenenfalls substituierte Phenylaminogruppe bedeutet, sowie deren pharmazeutisch akzeptable Säureadditionssalze, wobei für die Fälle, in denen für $R^2$ stehendes Alkyl durch eine Carbonsäuregruppierung substituiert ist, für R und $R^1$ Alkyl .stehen muß und wobei $R^3$ nicht für Wasserstoff stehen kann, wenn R und $R^1$ Wasserstoff bedeuten und der Pyrrolring in 3-Stellung formyliert ist.

Die Formylgruppe kann in der 2-oder 3-Position des Pyrrolrings stehen.

Die als Substituierten eines für $R^2$ stehenden Alkylrestes genannten Carbonsäuregruppierungen bedeuten insbesondere Amide, wie das unsubstituierte Amid, Mono-und Dialkylamide mit 1 bis 3 C-Atomen pro Alkylrest, Ester, wie der Methyl-oder Ethylester, die Nitrilgruppe oder die Carboxygruppe selbst.

Die für $R^3$ stehenden oder als Substituent an einen für $R^2$ stehenden Alkylrest gebundenen gegebenenfalls substituierten aliphatischen oder aromatischen 5-bis 7-gliedrigen Heterocyclen enthalten als Heteroglieder vorzugsweise $\geq$O, $\geq$S oder $\geq$NR$^4$. Sofern der Heterocyclus 2 Heteroglieder aufweist, können diese gleich oder verschieden sein. Ein Stickstoff enthaltender Heterocyclus kann auch über das Hetero-N-atom gebunden sein; er kann dann neben dem ersten, die Bindung vermittelnden Stickstoffatom noch ein beliebiges der oben genannten Heteroglieder enthalten. Beispiele fér deraritge, über ein Hetero-N-atom gebundene heterocyclische Reste sind der N-Pyrrolidinrest oder der N-Thiomorpholinrest.

Aromatische heterocyclische Reste sind solche, die aufgrund eine Konjugation von Doppelbindungen, gegebenenfalls mit freien Elektronenpaaren, innerhalb des Ringes mesomere Grenzstrukturen ausbilden können, wie z.B. der Thienylrest oder der Pyrazolylrest. Aliphatische heterocyclische Reste enthalten nur isolierte oder gar keine Doppelbindungen, wie z.B. der Pyrrolidinrest, der Piperidinrest, der Morpholinrest oder der Perhydrothiazepinrest. Von den zwei Heteroglieder enthaltenden Heterocyclen sind solche bevorzugt, die mindestens ein Stickstoff ehthaltendes Heteroglied aufweisen.

Beispiele für heterocyclen, von denen sich für $R^3$ stehende oder als Substituent an einen für $R^2$ stehenden Alkylrest gebundene heterocyclische Reste ableiten, sind: Thiophen, Di-oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, Imidazol, Imidazolin, Imidazolin, Oxazol, Oxazolin, Oxazolidin, Thiazol, Thiazolin, Thiazolidin, Pyrimidin, Pyridazin, Pyrazin, Piperazin, Morpholin, Thiomorpholin, Diazepin, Oxazepin, Thiazepin, Perhydrodiazepin, -oxazepin und-thiazepin.

Besonders bevorzugte heterocyclishe Rest leiten sich ab von Pyrrol, Pyrrolidin, Imidazol, Thiazol, Thiazolidin und Perhydrothiazepin.

Bei den Heterogliedern der Formel $\geq$N-R$^4$ steht $R^4$ für Wasserstoff, Alkyl mit 1 bis 4, vorzugsweise 1 oder 2, C-Atomen oder Alkoxycarbonyl mit 1 bis 4 C-Atomen in der Alkoxygruppe.

Die heterocyclischen Reste können auch an einem der Ringkohlenstoffatome einen Substituenten wie z.B. eine Carboxylgruppe, eine Formylgruppe, Alkoxycarbonyl mit 1 bis 4, vorzugsweise 1 oder 2, C-Atomen in der Alkoxygruppe oder, bevorzugt, eine Alkylgruppe mit 1 bis 4, vorzugsweise 1 oder 2, C-Atomen tragen.

Aliphatische heterocyclische Reste, insbesondere solche, die sich von Stickstoffheterocyclen ableiten, können auch an einem Ringkohlenstoffatom, vorzugsweise einem dem Stickstoff enthaltenden Heteroglied benachbarten Ringkohlenstoffatom, eine Ketofunktion, ein doppelt gebundenes Sauerstoffatom, aufweisen. Dieses kann auch in seiner tautomeren Form vorliegen.

Heterocyclische Reste, die als Substituent an eine für $R^2$ stehende Alkylgruppe gebunden sind, können gegebenenfalls an einen Benzolkern kondensiert sein, der seinerseits gegebenenfalls durch Alkyl mit 1 bis 4, vorzugsweise 1 oder 2 C-Atomen, oder Alkoxy mit 1 bis 4, vorzugsweise 1 oder 2 C-Atomen, substituiert sein kann. Beispiele für kondensierte Heterocyclen, von denen sich bevorzugte heterocyclische Reste ableiten, die als Substituent an eine für $R^2$ stehende Alkylgruppe gebunden sind, sind Indol und Chinazolin.

Ein für $R^3$ stehender Phenylrest und ein als Substituent an einer für $R^2$ oder $R^3$ stehenden Alkylgruppe gebundener Phenoxyrest kann im Kern seinerseits bis zu drei Substituenten tragen und zwar eine Aminogruppe, Monoalkylamino mit 1 bis 4, vorzugsweise 1 oder 2, C-Atomen, Dialkylamino mit insgesamt 2 bis 6, vorzugsweise 2 bis 4, C-Atomen, Alkanoylamino mit 1 bis 6, vorzugswiese 1 oder 2, C-Atomen, Alkyl mit 1 bis 4, vorzugsweise 1 oder 2, C-Atomen, Alkoxy mit 1 bis 4, vorzugsweise 1 oder 2, C-Atomen, Halogen, vorzugsweise Fluor, Chlor oder Brom, insbesondere Fluor oder Chlor, Hydroxy, Nitro, Cyano, Carboxy oder Alkoxycarbonyl mit 1 bis 4, vorzugsweise 1 oder 2, C-Atomen in der Alkoxygruppe. Als gegebenenfalls zweiter Substituent des Kerns kommen in Betracht: eine der oben definierten Alkyl-oder Alkoxygruppen oder eines der oben genannten Halogene und als dritter Substituent eine der obigen Alkyl-oder Alkoxygruppen.

Ein einzelner Substituent kann in 2-, 3-oder 4-Stellung des Phenyl-bzw. Phenoxykerns stehen, Bei Zweifachsubstitution sind von den möglichen Positionen die 2,4-, die 3,4-und die 3,5-Stellung bevorzugt. Bei der Dreifachsubstitution kommen die Positionen 2,3,4 und 3,4,5 und 2,4,6 in Betracht.

Bevorzugte Substituenten für die genannten Phenylkerne sind Chlor, Alkyl und Alkoxy mit 1 oder 2 C-Atomen, insbesondere Methyl oder Methoxy, Carboxy und Alkoxycarbonyl mit 1 oder 2 C-Atomen in der Alkoxygruppe. Bevorzugt sind ferner neben den unsubstituierten Kernen die monosubstituierten und disubstituierten, welche als zweiten Substituenten einen Alkoxyrest tragen.

Für die Anzahl und Stellung der Substituenten im Phenylkern einer für $R^3$ stehenden, gegebenenfalls

substituierten Phenylaminogruppe gelten, eingeschränkt auf die heir möglichen Substituenten, die gleichen Bedingungen wie für die oben beschriebenen Phenyl-und Phenoxyreste.

In einem an die Piperazingruppe gebundenen Toluyl-, Chlorophenyl-oder Alkoxyphenylrest können die Substituenten, bezogen auf den Piperazinrest, in 2-, 3-oder 4-Stellung, vorzugsweise in 2-oder 4-Stellung, stehen.

Spezielle, besonders bevorzugte Reste $R^2$ sind Alkylreste mit 1 bis 3 C-Atomen, die durch einen der folgenden Substituenten substituiert sind:
Formylamino, Acetylamino, Propionylamino, Isopropionylamino, Butyrylamino, 4-Chlorophenoxyacetylamino, (2-Oxopyrrolidin-1-yl)-acetylamino, N,N-Dimethylamin-acetylamino, L-Thiazolidin-4-yl-carbonylamino, 4-Chlorobenzoyl amino, 5-Oxoperhydro-(1,4)-thiazepin-3-yl-carbonylamino, Aminocarbonylamino, 4-Chlorophenylaminocarbonylamino, 1-Acetyl-L-pyrrolidin-2-yl-carbonylamino, 1-Ethyl(pyrrolidin-2-yl), 2-Oxopyrrolidin-1-yl, Imidazol-4-yl, (2,5-Dimethyl-3-formyl)-pyrrol-1-yl, Indol-3-yl.

Weitere wertvolle Substituenten eines für $R^2$ stehenden Alkylrestes mit 1 bis 3 C-Atomen sind z.B.: die Aminogruppe, 3,4-Dimethoxybenzoylamino, 2-(4-(2-Methoxyphenyl)-piperazinyl)-acetylamino, Isobutyrylamino, 3-Tert. butoxycarbonyl-thiazolidin-4-yl-carbonylamino, 4-Ethoxycarbonylphenylamino-carbonylamino, 4-Ethoxycarbonylphenylamino-thiocarbonylamino, 4-Carboxyphenylamino-carbonylamino, 4-Carboxyphenylamino-thiocarbonylamino, Thiazolidin-4-yl, 2-Methylpyrrol-4-yl.

Die Herstellung der erfindungsgemäßen Verbindungen der allgemeinen Formel I kann beispielsweise durch Formylierung entsprechender Pyrrole der allgemeinen Formel III

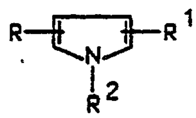

(III)

erfolgen. Derartige Pyrrole sind zum Beispiel in der DE-OS 35 27 791.2 beschreiben.

Als Formylierungsreagenzien eignen sich viele in der Literatur beschriebenen Methoden (s. Houben-Weyl, Methoden der Organischen Chemie, 4. Auflage, E3, S. 16 ff. (1983)). In speziellen Fällen ist die Variante nach Reimer-Tiemann durch Umsetzung der Pyrrole mit Chloroform im alkalischen Medium angebracht, bessere Ausbeuten ergeben jedoch die Umsetzungen der Pyrrole mit 1,1-Dihalogenethern und Friedel-Crafts-Katalysatoren (J. Med. Chem. 15, 97 (1972)) oder mit Trialkoxymethanen und Trifluoressigsäure (J. Org. Chem. 43, 283 (1978)). Am einfachsten führt die Vilsmeier-Haack-Reaktion durch Umsetzung der Pyrrole der allgemeinen Formel III mit Formamiden und Phosphoroxychlorid zu den erfindungsgemäßen Verbindungen ( Methodicum Chimicum 5, S. 234 (1975), J. Org. Chem. 28, 3052 (1963)). Phosphoroxychlorid kann durch andere Verbindungen wie Oxalylchlorid, Thionylchlorid, Sulfurylchlorid oder Cyanurchlorid ersetzt werden. Vorteilhaft wird die Reaktion in Gegenwart eines Lösungsmittels wie Dimethylformamid, 1,2-Dichlorethan oder Ethern durchgeführt. Auch Isonitrile in saurer Lösung eignen sich als Formylierungsagenzien für die erfindungsgemäßen Verbindungen (Chem. Ber. 94, 298 (1961)).

Die als Vorstufen benötigten Pyrrole der allgemeinen Formel III können entweder durch Umsetzung entsprechend substituierter Furane mit Aminen der allgemeinen Formel IV

$H_2N$-$R^2$ (IV)

(analog US 2,655,512) oder durch Umsetzung von 1,4-Dicarbonylverbindungen mit Aminen der Formel IV (vergl. DE-OS 35 27 791.2) erfolgen.

Die entsprechenden Aldehyde können aber auch direkt analog US 2,655,512 aus den Furanaldehyden und den entsprechenden Aminen erhalten werden.

Die Umsetzungen werden bevorzugt unterhalb oder bei der Siedetemperatur eines geeigneten Lösungsmittels ausgeführt. Gewünschtenfalls, z.B. bei Einsatz besonders niedrig siedender Lösungsmittel kann die Umsetzung auch unter Druck oberhalb des Siedepunktes des Reaktionsgemisches ausgeführt werden.

Geeignete Lösungsmittel sind z.B. Alkohole, insbesondere solche mit 1 bis 6 C-Atomen, wie z.B. Methanol, Ethanol; i-und n-Propanol, i-, sec-und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylether, Methylethylether, Di-n-propylether, Diisopropylether, Methyl-n-Butylether, Ethylpropylether, Dibutylether, Tetrahydrofuran, 1,4-Dioxan, 1,2-Dimethoxyethan, Bis-$\beta$-methoxyethylether; Polyether, wie z.B. Polyethylenglykole mit einem Molekulargewicht bis ca. 600; Oligoethylen-glykol-dimelthylether, wie z.B. Pentaglyme; aliphatische Carbonsäuren, insbesondere Ameisen-und Essigsäure; Glykole und teilweise veretherte Glykole, wie z.B. Ethylenglykol, Propylenglykol, Trimethylenglykol, Ethylenglykol-monomethylether,

Ethylenglykol-monoethylether, Diethylenglykol-monoethylether; aliphatische Kohlenwasserstoffe, wie z.B. niedrigund hochsiedende Petrolether; aromatische Kohlenwasserstoffe, wie z.B. Benzol, Toluol, o-, m-und p-Xylol; halogenierte aliphatische oder aromatische Kohlenwasserstoffe, wie z.B. Melthylenchlorid, Chloroform, Tetrachlorkohlenstoff, Chlorbenzol, Dichlorbenzol; Nitrile, wie z.B. Acetonitril; Amide, wie z.B. Dimethylformamid, N-Methyl-pyrrolidon; Hexamethylphosphorsäuretriamid; Sulfoxide, wie z.B. Dimethylsulfoxid; Wasser. Auch Gemische verschiedener Lösungsmittel können verwendet werden.

Das Amin der allgemeinen Formel IV kann auch in Form eines Säureadditionssalzes eingesetzt werden. Die Aufarbeitung der Ansätze erfolgt nach üblichen Methoden. Die Umsetzung kann gegebenenfalls auch in Anwesenheit einer Base oder eines Basengemisches durchgeführt werden. Geeignete Basen sind z.B. tertiäre aliphatische Amine, wie z.B. Triethylamin, Tri-n-propylamin und Tri-iso-propyl amin, ferner Pyridin, sowie Alkali-carbonate, -hydrogen-carbonate.

Durch Abspaltung des Acylrestes aus erfindungsgemäßen Verbindungen der allgemeinen Formel I, in denen $R^2$ ein durch die Gruppe der allgemeinen Formel II

$$-NH-\overset{X}{\overset{\|}{C}}-R^3 \quad (II)$$

substituierter Alkylrest ist, können die entsprechenden Verbindungen, in denen der für $R^2$ stehende Alkylrest durch eine primäre Aminogruppe substituiert ist, erhalten werden. Die Abspaltung wird in an sich bekannter Weise hydrolytische durchgeführt. Hierzu werden die Verbindungen mit Wasser oder einem wasserhaltigen organischen Medium in Gegenwart von molaren Mengen einer Base behandelt. Die Behandlungsdauer hängt von der gewählten Temperatur ab. Man kann bei Zimmertemperatur oder, um die Hydrolyse zu beschleunigen, bei erhöhter Temperatur, zweckmäßigerweise bis zur Rückflußtemperatur des flüssigen Hydrolysemediums, arbeiten.

Die Art der zuzusetzenden Base ist im Prinzip belanglos. Diese Reagentien. sollen nur eine ausreichend hohe $OH^-$-Konzentration herbeiführen und eine einfache Aufarbeitung der Ansätze gestatten. Die Wahl dieser Mittel kann daher in bekannter Weise erfolgen.

Zur Herstellung der erfindungsgemäßen Verbindungen der allgemeinen Formel I, in denen $R^2$ ein durch eine Acylaminogruppe der allgemeinen Formel II

$$-NH-\overset{X}{\overset{\|}{C}}-R^3 \quad (II)$$

substituierter Alkylrest ist, können auch Aminoalkylpyrrolaldehyde der allgemeinen Formel V

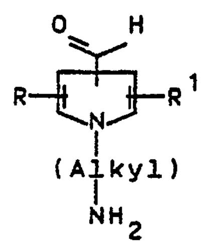

(V)

mit reaktiven Carbonsäurederivaten, abgeleitet von Carbonsäuren der allgemeinen Formel VI

$R^3$-COOH (VI)

worin $R^3$ die oben angegebene Bedeutung hat oder auch mit Alkalicyanat bzw. -thiocyanat oder mit Isocyanaten bzw. Isothiocyanaten der allgemeinen Formel VII

$R^5$-NCX (VII)

worin X Sauerstoff oder Schwefel ist und $R^5$ Phenyl oder Alkyl bedeutet, das gegebenenfalls in der oben präzisierten Weise substituiert ist, acyliert werden.

Als reaktive Carbonsäurederivate eignen sich Carbonsäureester, carbonsäureanhydride, Carbonsäurechloride oder Carbonsäuren, die in situ aktiviert werden, wie z.B. mit Dicyclohexylcarbodiimid (Houben Weyl 8, 522), Oxalylchlorid (GB 2139-225), N,N-Carbonyldiimidazol (J.Med.chem.1982; 620, Synthesis 1982, 833; Chem. Pharm. Bull. 32, 5044 (1984)); N,N'-Carbonyldiazolen (Bull. Chem. Soc. Jap. 57, 3597 (1984)); Kohlensäure-di-(2-pyridyl)-ester (Tetrahedron Lett. 25, 4943 (1983)); Chlorameisensäureester (Tetrahedron Lett. 24, 3365 (1983)); Diphosphortetrajodid (Chem. Lett. 1983, 449); Dialkyldisulfit (Indian J. Chem. 21. 259 (1982)); Methylethylphosphinsäureanhydrid oder mit anderen reaktiven Agenzien.

Bei Einsatz von Isocyanaten bzw. Isothiocyanaten der allgemeinen Formeln $R^5$-NCO bzw. $R^5$-NCS als Acylierungsmittel werden diejenigen erfindungsgemäßen Pyrrolderivate erhalten, in denen $R^2$ ein durch die Gruppen -HN-CO-NH-$R^5$ bzw. -NH-CS-NH-$R^5$ substituierter Alkylrest ist. Umsetzung der Amine der allgemeinen Formel V mit Alkalicyanaten bzw. -thiocyanaten liefert erfindungsgemäße Verbindungen, in denen

$R^2$ ein durch die Gruppen -NH-CO-$NH_2$ bzw. -NH-CS-$NH_2$ substituierter Alkylrest ist.

Die Umsetzungen werden zweckmäßigerweise in flüssiger Phase durchgeführt, wobei die Anwesenheit eines inerten Lösungsmittels vorteilhaft ist.

Werden enantiomerenreine Carbonsäurederivate oder Amine der allgemeinen Formel V eingesetzt, so können auch die erfindungsgemäßen Verbindungen der allgemeinen Formel I als enantiomerenreine Verbindungen erhalten werden.

Eine andere Möglichkeit zur Herstellung der erfindungsgemäßen Verbindungen geht von 2,5-Dialkoxytetrahydrofuranaldehyden der allgemeinen Formel VIII aus, die mit Aminen der allgemeinen Formel IV unter Säurekatalyse direkt zu entsprechend substituierten Pyrrolaldehyden der allgemeinen Formel I umgesetzt werden können (vergl. Synthesis 1973, 422).

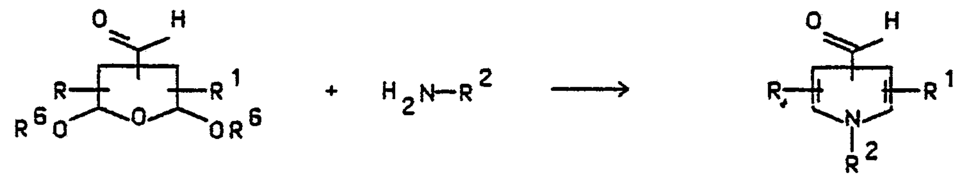

(VIII) (IV) (I)

$R^6$ bedeutet niedriges Alkyl mit 1 bis 4 C-Atomen.

Analog dieser Methode können auch Verbindungen der allgemeinen Formel III synthetisiert werden (Synthesis 1981, S. 482), die dann nach den vorstehend beschriebenen Verfahren formyliert werden können.

Sofern die erfindungsgemäßen Verbindungen der allgemeinen Formel I basische Reste enthalten, bilden sie mit anorganischen oder organischen Säuren Säureadditionssalze. Zur Bildung derartiger Säureadditionssalze sind anorganische und organische Säuren geeignet. Geeignete Säuren sind - beispeilsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Äpfel-, Sulfamin-, Phenylpropion-, Glucon-, Ascorbin-, Nicotin-, Isonicotin-, Methansulfon-, p-Toluolsulfon-, Zitronen-oder Adipin-Säure. Pharmakologisch annehmbare Säureadditionssalze werden bevorzugt. Die Säureadditionssalze werden wie üblich durch Vereinigung der Komponenten, zweckmäßigerweise in einem geeigneten Lösungsmittel oder Verdünnungsmittel, hergestellt. Bei der Synthese der Verbindungen der Formel I können zunächst die Säureadditionssalze im Zuge der Aufarbeitung anfallen. Aus den Säureadditionssalzen können die freien Verbindung der allgemeinen Formel I gewünschtenfalls in bekannter Weise, z.B. durch Auflösen oder Suspendieren in Wasser und Alkalischstellen, z.B. mit Natronlauge, und anschließendes Isolieren, gewonnen werden.

Die erfindungsgemäßen Verbindungen der allgemeinen Formel I und ihre pharmazeutisch akzeptablen Säureadditionssalze besitzen wertvolle pharmakologische Eigenschaften. Sie sind zentralwirksam, beispielsweise zeigen sie encephalotrope und nootrope Wirkungen und dienen zur Behandlung von Krankheiten der Gehirnfunktionen wie cerebraler Insuffizienz, cerebraler Alterungsvorgänge, verminderter Gedächtnisleistung wie sie auch bei der Alzheimer Krankheit oder der Multi-Infarkt Demenz oder bei verminderter Lernleistung auftreten. Sie sind den bisher bekannten Verbindungen gleicher Wirksrichtung überraschenderweise erheblich überlegen. Sie zeigen eine ausgezeichnete Wirksamkeit in verschiedenenartigen Tests, wie z.B. bei der Verlängerung der Überlebenszeit unter Natriumnitrit-Hypoxie nach Gibsen und Bless (J. Neurochemistry27, (1976)), bei der Verbesserung der stickstoffinduzierten Hypoxietoleranz, wobei Versuchstiere nach Prämedikation mit den untersuchten Präparaten mit reinem Stickstoff beatmet werden und die Verlängerung des Zeitraumes zwischen Einsatz der Beatmung und elektrischer Neutralität des Elektroencephalogramms sowie die Letalität gemessen werden.

Auch in Tests, die direkt auf die Erfassung der Lern-und Gedächtnisleistung abzielen, wie z.B. den bekannten "avoidance"-Tests, sind die erfindungsgemäßen Produkte sehr gut wirksam.

Die Prüfung in den genannten und einer Reihe weiterer Tests, wie z.B. dem $\gamma$-Butyrolacton-Test, ergibt, daß die erfindungsgemäßen Verbindungen in niedrigen Dosen bei geringer Toxizität überraschenderweise ein besonders günstiges, bei bekannten Präparaten in dieser Form nicht vorliegendes Wirkprofil aufweisen.

Die Verbindungen der allgemeinen Formel I und ihre physiologisch verträglichen Salze stellen somit

eine Bereicherung der Pharmazie dar.

Ferner wurde gefunden, daß auch die bereits bekannten Verbindungen der allgemeinen Formel I, bei denen für $R^2$ stehendes Alkyl durch eine Carbonsäuregruppierung substituiert ist und R und/oder $R^1$ Wasserstoff bedeuten (siehe z.B. V. Carelli et al, Ann.Chim. Appli. 53, 309 (1963); A. Nudelman et al, J. Med. Chem.21, 962 (1978); K.Olsson et al, Acta Chem. Scand. Ser.B, 33, 125 (1979)) sowie die bereits bekannten Verbindungen der Formel I, bei denen $R^3$ und R und $R^1$ für Wasserstoff stehen und der Pyrrolring in 3-Stellung formyliert ist (DE-A 3531504), die gleichen, zentralwirksamen pharmakologischen Eigenschaften besitzen. Auch diese Verbindungen konnen nach den oben beschriebenen Synthesemethoden hergestellt werden.

Die erfindungsgemäßen Verbindungen der allgemeinen Formel I und die vorganannten Verbindung und ihre pharmazeutisch akzeptablen Säureadditionssalze könne daher am Menschen als Heilmittel z.B. bei der Bekämpfung bzw. Vorbeugung von Erkrankungen, die durch eine Einschränkung der Gehirnfunktion bedingt sind und bei der Behandlung und Vorbeugung von cerebralen Alterungsvorgängen Anwendung finden.

Die Verbindungen der allgemeinen Formel I und die vorgenannten Verbindungen und ihre pharmazeutisch akzeptablen Säureadditionssalze können als Heilmittel für sich allein, in Mischungen untereinander oder in Form von pharmazeutischen Zubereitung verabreicht werden, die eine enterale oder parenterale Anwendung gestatten und die als aktiven Bestandteil eine wirksame Dosis mindestens einer Verbindung der allgemeinen Formel I bzw. der vorgenannten Verbindungen oder eines Säureadditionssalzes davon, neben üblichen pharmazeutisch einwandfreien Träger-und Zusatzstoffen enthalten. Die Zubereitungen enthalten normalerweise etwa 0,5 bis 90 Gew.% der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organisch Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen sind Sirupen eignen sich z.B. Wasser, Saccharose, Invertzucker, Glukose, Polyole etc. Als Trägerstoffe für die Herstellung von Injektionslösungen einen sich z.B. Wasser, Alkohole, Glycerine, Polyole, pflanzliche Öle etc.

Die pharmazeutischen Präparate können neben den Wirk-und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Ge schmacks-oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch einen oder mehrere andere therapeutische wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbocromen, Dipyridamol, Nifedipin und Perhexilin, antianginöse Verbindungen, wie Isosorbiddinitrat, Iosorbidmonomitrat, Glycerolnitrat, Molsidomin und Verapamil, β-Blocker, wie Propranolol, Oxprenolol, Atenolol, Metoprolol und Penbutolol. Darüberhinaus lassen sich die Verbindungen mit anderen noottrop wirksamen Substanzen, wie z.B. Piracetam, oder ZNSaktiven Substanzen, wie Pirlindol, Sulpirid etc. kombinieren.

Die Dosierung kann innerhalb weiter Grenzen varriieren und ist in jedem einzelnen Fall den individuellen Gegebenenheiten anzupassen. In allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Application beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z.B. 2, 3 oder 4, Teilverabreichungen aufgeteilt. Gegebenenfalls kann es, je nach individuallem Verhalten, erforderlich werden, von der angegebenen Tagesdosis nach oben oder nach unten abzuweichen. Pharmazeutische Präparate enthalten normalerweise 0,1 bis 50 mg, vorzugsweise 0,5 bis 10 mg Wirkstoff der allgemeinen Formel I oder eines pharmakologisch annehmbaren Salzes davon pro Dosis.

Die folgenden Beispiele 1 bis 15 betreffen die Herstellung von Verbindungen der allgemeinen Formel I, die Beispiele A bis H betreffen die Herstellung von Zubereitungen der Verbindungen der allgemeinen Formel I.

Beispiel 1:

1-(2-Acetylaminoethyl)-2,5-dimethylpyrrol-3-aldehyd

Zu 13 ml (0,168 mol) wasserfreiem Dimethylformamid werden bei 0°C langsam 15,5 ml (0,166 mol) Phosphoroxychlorid zugetropft. Man rührt 15 min bei 10°C, gibt 70 ml 1,2-Dichlorethan zu und tropft anschließend 29,4 g (0,163 mol) 1-(2-Acetylaminoethyl)-2,5-dimethylpyrrol in 25 ml 1,2-Dichlorethan bei 5°C. Man rührt 6 h bei Raumtemperatur, gibt 79,5 g (0,97 mol) Natriumacetat in 130 ml Wasser zu, erhitzt 15 min unter Rückfluß, trennt die Phasen und engt die organische Phase ein.

Der Rückstand wird mit Toluol ausgekocht und das ausfallend Produkt abfiltriert und aus Essigester umkristallisiert.
Ausbeute: 17,7 g (52 % d. Th.)
Schmelzpunkt: 119 - 120°C
Elementaranalyse: $C_{11}H_{16}N_2O_2$ (208,26)
Berechnet: C 63,4 H 7,7 N 13,5 O 15,4
Gefunden: C 63,3 H 7,7 N 13,5 O 15,4 Analog Beispiel 1 lassen sich herstellen:

Beispiel 2:
Ausgehend von 1-(2-Acetylaminoethyl)-5-methyl-pyrrol:
1-(2-Acetylaminoethyl)-5-methyl-pyrrol-2-aldehyd
Schmelzpunkt: 87-90°C

Beispiel 3:
Ausgehend von 1-(2-(4(5)-Imidazolyl)-ethyl)-2,5-dimethyl-pyrrol:
1-(2-(4-(5)-Imidazolyl)-ethyl)-2,5-dimethyl-pyrrol-3-aldehyd
Schmelzpunkt: 157-160°C

Beispiel 4:
Ausgehend von 1-(3-Acetylaminopropyl)-2,5-dimethyl-pyrrol:
1-(3-Acetylaminopropyl)-2,5-dimethyl-pyrrol-3-aldehyd

Beispiel 5:
Ausgehend von 1-(2-Butyrylaminoethyl)-2,5-dimethyl-pyrrol:
1-(2-Butyrylaminoethyl)-2,5-dimethyl-pyrrol-3-aldehyd
Schmelzpunkt: 75-76°C

Beispiel 6:
Ausgehend von 1-(2-Acetylaminoethyl)-pyrrol:
1-(2-Acetylaminoethyl)-pyrrol-2-aldehyd
Schmelzpunkt: 60-61°C

Beispiel 7:
Ausgehend von 1-(2-(Pyrrolidin-2-on-1-yl)ethyl)2,5-dimethyl-pyrrol:
1-(2-(Pyrrolidin-2-on-1-yl)ethyl)-2,5-dimethyl-pyrrol-3-aldehyd
Schmelzpunkt: 122-124,5°C

Beispiel 8:
Ausgehend von 2,5-Dimethyl-1-(aminocarbonylmethyl)-pyrrol:
2,5-Dimethyl-1-(aminocarbonylmethyl)-pyrrol-3-aldehyd
Schmelzpunkt: 180-181°C

Beispiel 9:

1,2-Di-(2,5-dimethyl-3-formyl-pyrrol-1-yl)-ethan

Zu 7,2 ml (0,093 mol) wasserfreiem Dimethylformamid werden bei 0°C langsam 8,7 ml (0,093 mol) Phosphoroxychlorid zugetropft. Man rührt 15 min bei 10°C, gibt 10 ml 1,2-Di-chlorethan zu und tropft bei 5°C 10 g (0,046 mol) 1,2-Di-(2,5-dimethyl-pyrrol-1-yl)-ethan in 40 ml 1,2-Dichlorethan zu. Nach 15 h Reaktionszeit bei Raumtemperatur werden 100 g Natriumacetat in 150 ml Wasser zugesetzt, 5 min unter Rückfluß erhitzt, mit Methylenchlorid versetzt, die Phasen getrennt, die organische Phase eingeengt und aus Isopropanol umkristallisiert.
Ausbeute: 5,5 g (44 % d. Th.)
Schmelzpunkt: 225 - 227°C
Elementaranalyse: $C_{16}H_{20}N_2O_2$ (272,35)
Berechnet: C 70,6 H 7,4 N 10,3 O 11,7
Gefunden: C 71,1 H 7,7 N 10,3 O 11,5

Beispiel 10:

2,5-Dimethyl-1-(methoxycarbonylmethyl)-pyrrol-3-aldehyd

17 ml (0,18 mol) Phosphoroxychlorid, 14 ml (0,18 mol) wasserfreies Dimethylformamid und 30 g (0,18 mol) (2,5-Dimethyl-pyrrol-1-yl)-essigsäuremethylester werden in 140 ml 1,2-Dichlorethan wie oben beschrieben umgesetzt.
Ausbeute: 16,2 g (46 % d. Th.)
Schmelzpunkt: 83 - 85°C
Elementaranalyse: $C_{10}H_{13}NO_3$ (195.22)
Berechnet: C 61,5 H 6,7 N 7,2 O 24,6
Gefunden: C 61,8 H 6,9 N 7,1 O 24,8

Beispiel 11:

1-(2-Acetylaminoethyl)-pyrrol-3-aldehyd

8,5 g (0,053 mol) 2,5-Dimethoxytetrahydrofuran-3-aldehyd und 5,4 g (0,053 mol) 2-(Acetylamino)-ethylamin werden 1 h bei 60°C gerührt. Nach Zugabe von 0,05 g p-Toluolsulfonsäurehydrat rührt man 90 min bei 80°C, kühlt ab, versetzt mit Natriumhydrogencarbonatlösung und extrahiert mit Methylenchlorid, dann mit Pentanol. Die organischen Phasen werden eingeengt und über eine Kieselgelsäule mit Methylenchlorid/Methanol = 95:5 als Laufmittel chromatographiert. Das isolierte Produkt wird aus

Essigester/Ligroin kristallisiert.
Ausbeute: 2,3 g (24 % d. Th.)
Schmelzpunkt: 58 - 60°C
Elementaranalyse: $C_9\ H_{12}N_2O_2$ (180,21)
Berechnet: C 60,0 H 6,7 N 15,5 O 17,8
Gefunden: C 59,5 H 6,8 N 15,3 O 18,3

Beispiel 12

1-(2-Dimethylaminoethyl)-2,5-dimethylpyrrol-3-aldehydtartrat

Zu 8,2 ml (0,106 mol) wasserfreiem Dimethylformamid werden bei 0°C 9,8 ml; (0,107 mol) Phosphoroxychlorid zugetropft. Man rührt 15 min bei 10°C, gibt 20 ml 1,2-Dichlorethan zu und tropft bei 5°C 17,1 g (0;103 mol) 2,5-Dimethyl-1-(2-dimethylamino)-pyrrol in 60 ml 1,2-Dichlorethan zu. Man rührt 20 Stunden bei Raumtemperatur, gibt 50 g Natriumacetat in 100 ml Wasser zu, erhitzt 15 min unter Rückfluß, extrahiert nach dem Abkühlen mit Methylenchlorid und engt ein. Nach Chromatographie über eine Kieselgelsäure (Laufmittel : Methylenchlorid : Methanol = 99 : 1 bis 96 : 11) wird in Isopropanol gelöst, mit Weinsäure versetzt, der Niederschlag abgesaugt und getrocknet.
Ausbeute: g 10,1 (28 % d. Th.)
Schmelzpunkt: 171 - 173°C
Elementaranalyse: $C_{15}\ H_{24}N_2O_7$ (344,36)
Berechnet: C 52,3 H 7,0 N 8,1 O 32,5
Gefunden: C 52,1 H 6,7 N 8,0 O 32,5

Beispiel 13
1-(Aminocarbonylmethyl)-2,5-dimethylpyrrol-3-aldehyd
Schmelzpunkt: 217 - 220°C

Beispiel 14
1-(2-Benzoylaminoethyl)-2,5-dimethyl-pyrrol-3-aldehyd
Schmelzpunkt: 119 - 120°C

Beispiel 15
1-(2-(Indol-3-yl)-ethyl)-2,5-dimethyl-pyrrol-3-aldehyd
Schmelzpunkt 151 - 153°C

Beispiel A

Emulsionen mit 3 mg Wirkstoff per 5 ml können nach folgender Rezeptur hergestellt werden:
Wirkstoff 0.06g
Neutralöl q.s.
Natriumcarboxymethylzellulose 0,6g
Polyoxyethylenstearat q.s.
Reinglyzerin 0,2 bis 2g
Aromastoffe q.s.
Wasser (entmineralisiert oder destilliert) ad 100ml

Beispiel B

Tabletten können nach folgender Formulierung hergestellt werden:

| | |
|---|---|
| Wirkstoff | 2mg |
| Lactose | 60mg |
| Maisstärke | 30mg |
| lösliche Stärke | 4mg |
| Magnesiumstearat | 4mg |
| | 100mg |

Beispiel C

Für die Herstellung von Weichgelatinekapseln mit 5 mg Wirkstoff pro Kapsel eignet sich die folgende Zusammensetzung:

| | |
|---|---|
| Wirkstoff | 5mg |
| Mischung von Triglyzeriden aus Kokosöl | 150mg |
| Kapselinhalt | 155mg |

Beispiel D

Für die Herstellung von Dragees eignet sich folgende Formulierung

| | |
|---|---|
| Wirkstoff | 3mg |
| Maisstärke | 100mg |
| Lactose | 55mg |
| sec Calciumphosphat | 30mg |
| lösliche Stärke | 3mg |
| Magnesiumstearat | 5mg |
| kolloidale Kieselsäure | 4mg |
| | 200mg |

Beispiel E

Dragees, enthaltend einen erfindungsgemäßen Wirkstoff und einen anderen therapeutisch wirksamen Stoff:

| | |
|---|---|
| Wirkstoff | 6mg |
| Propranolol | 40mg |
| Milchzucker | 90mg |
| Maisstärke | 90mg |
| sec Calciumphosphat | 34mg |
| lösliche Stärke | 3mg |
| Magnesiumstearat | 3mg |
| kolloidale Kieselsäure | 4mg |
| | 270mg |

Beispiel F

Dragees, enthaltend einen erfindungsgemäßen Wirkstoff und einen anderen therapeutisch wirksamen Stoff:

| | |
|---|---|
| Wirkstoff | 5mg |
| Pirlindol | 5mg |
| Milchzucker | 60mg |
| Maisstärke | 90mg |
| sec Calciumphosphat | 30mg |
| lösliche Stärke | 3mg |
| Magnesiumstearat | 3mg |
| kolloidale Kieselsäure | 4mg |
| | 200mg |

Beispiel G

Kapseln, enthaltend einen erfinfungsgemäßen Wirkstoff und einen anderen therapeutisch wirksamen Stoff:

| | |
|---|---|
| Wirkstoff | 5mg |
| Nicergolin | 5mg |
| Maisstärke | 185mg |
| | 195mg |

Beispiel H

Injektionslösungen mit 1mg Wirkstoff pro ml können nach folgender Rezeptur hergestellt werden:

| | |
|---|---|
| Wirkstoff | 1,0mg |
| Polyethylenglykol 400 | 0,3mg |
| Natriumchlorid | 2,7mg |
| Wasser zu Injektionszwecken auf | 1 ml |

Bei der pharmakologischen Prüfung wurden z.B. folgende Ergebnisse erhalten:
Nitrit-Hypoxie bei der Maus
In diesem Test wird nach der Methode von Gibson und Blass (J.Neurochem. 27, 37 (1976) bei Mäusen mit $NaNO_2$ (175 mg/kg s.c.) eine cerebrale Hypoxie erzeugt, die zu mas siven Verhaltensströungen der Tiere führt. Bestimmt wird, ob die Haltefähigkeit an einem Drehstab durch Praemedikation mit der Testsubstanz beeinflußt wird. Die erfindungsgemäßen Verbindungen werden dabei in einer Dosis von 3 mg/kg per os Verabreicht. Die Ergebnisse sind in Tabelle 1 angegeben.

## Tabelle 1

Prozentuale Umkehr der Störung der Haltefähigkeit nach Verabreichung von 175 mg/kg s.c. $NaNO_2$ und Praemedikation mit den Verbindungen der allgemeinen Formel I.

| Verbindung der allgemeinen Formel I gemäß Beispiel | Prozentuale Umkehr des Hypoxie-Effektes |
|---|---|
| Nr. 1 | 67 |
| 4 | 46 |
| 5 | 23 |
| 6 | 66 |
| 7 | 18 |
| 8 | 51 |
| 9 | 36 |
| 10 | 72 |
| 11 | 73 |
| 12 | 31 |
| 13 | 44 |
| Piracetam (Vergleich mit 10 mg/kg | 19 |
| 100 mg/kg | 58 |

"Passive avoidance"

Die Testapparatur ist eine Hell-Dunkel-Box mit elektrifizierbarem Gitterboden im dunklen Teil.

55 Minuten nach Verabreichung von Kontroll-und Präparatinjektion werden unerfahrene männliche Mäuse mit Scopolaminhydrobromid (3 mg/kg i.p.) behendelt. 5 Minuten später werden die Mäuse in den hellen Teil der Box gesetzt. Nach dem Überwechseln in den drunklen Teil der Box erhalten sie einen ihnen unangenehmen elektrischen Fußschock. Nach 24 Stunden wird jede Maus einmal in den hellen Teil der Testapparatur gesetzt und die Verweildauer (max. 180 sec.) gemessen. Die mit einer aktiven Dosis eines Präparats und Scopolamin behandelten Tiere zeigen eine lange Verweildauer, ebenso die nicht mit Scopolamin behandelten Tiere, wogegen diejenigen mit Kontrollinjektion und Scopolamin behandelten eine kurze Verweildauer zeigen. Die erfindungsgemäßen Verbindungen werden dabei in einer Dosis von 3 bis 30 mg/kg p.o. verabreicht. Die Ergebnisse sind in Tabelle 2 angegeben. Bei diesem Test erzielt die bekannte verbindung Piracetam in einer Dosis von 60 mg/kg p.o. eine prozentuale Abschwächung von 100%.

## Tabelle 2

Prozentuale Abschwächung der scopolamininduzierten Amnesie, erkennbar an einer Verlängerung der Zeit bis zum Betreten des dunklen Teils des Passive-Avoidance Testraumes.

| Verbindung der allgemeinen Formel I gemäß Beispiel Nr. | Dosis (mg/kg) p.o. | Prozentuale Abschwächung |
|---|---|---|
| 1 | 3 | 219 |
| 4 | 30 | 92 |
| 5 | 30 | 96 |
| 6 | 10 | 63 |
| 9 | 10 | 297 |
| 10 | 3 | 108 |
| 11 | 3 | 126 |
| 12 | 30 | 128 |
| 13 | 30 | 146 |
| 14 | 30 | 124 |
| 15 | 30 | 98 |
| Piracetam (Vergleich) | 30 | 18 |

**Ansprüche**

1. Pyrrolaldehyde der allgemeinen Formel I

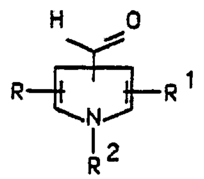

(I)

worin R und $R^1$ unabhängig voneinander Wasserstoff oder Alkyl mit 1 bis 4 C-Atomen,
$R^2$ Alkyl, das durch $-NH_2$, Acylamino der allgemeinen Formel II

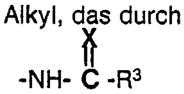

(II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und
$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Phenylaminogruppe bedeutet, sowie deren pharmazeutisch akzeptable Säureadditionssalze, wobei für die Fälle, in denen für $R^2$ stehendes Alkyl durch eine Carbonsäuregruppierung substituiert ist, für R und $R^1$ Alkyl stehen muß und wobei $R^3$ nicht für Wasserstoff stehen kann, wenn R und $R^1$ Wasserstoff bedeuten und der Pyrrolring in 3-Stellung formyliert ist.

2. Pyrrolaldehyde gemäß Anspruch 1 der allgemeinen Formel I

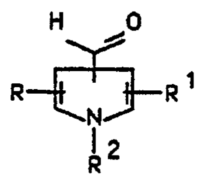

(I)

worin R und $R^1$ unabhängig voneinander Wasserstoff oder Alkyl mit 1 bis 4 C-Atomen,
$R^2$ Alkyl mit 1 bis 3 C-Atomen, das durch $-NH_2$, Acylamino der allgemeinen Formel II

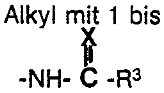

(II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeutet,
X für ein Sauerstoff-oder Schwefelatom steht und
$R^3$ Wasserstoff, Alkyl oder Alkylamino mit 1 bis 5, vorzugsweise 1 oder 2, C-Atomen, das gegebenenfalls durch $-NH_2$, Monoalkylamino mit 1 bis 4, vorzugsweise 1 oder 2, C-Atomen, Dialkylamino mit insgesamt 2 bis 6, vorzugsweise 2 bis 4, C-Atomen, einen N-Pyrrolidinyl-, N-Piperidinyl-, N-Morpholinyl-, N-Thiomorpholinyl-oder durch einen in 4-Stellung gegebenenfalls durch Alkyl mit 1 bis 4 C-Atomen, Phenyl, Toluyl, Chlorphenyl oder Methoxy-oder Ethoxyphenyl substituierten Piperazin-1-yl-Rest, durch Alkoxy mit 1 bis 4, vorzugsweise 1 oder 2, C-Atomen oder durch gegebenenfalls substituierts Phenoxy substituiert ist; Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Alkyl-oder Phenylaminogruppe bedeutet, wobei für die Fälle, in denen für $R^2$ stehendes Alkyl durch eine Carbonsäuregruppierung substituiert ist, für R und $R^1$ Alkyl stehen muß und wobei $R^3$ nicht für Wasserstoff stehen kann, wenn R und $R^1$ Wasserstoff bedeuten und der Pyrrolring in 3-Stellung formyliert ist.

3. Pyrrolaldehyde gemäß den Ansprüchen 1 und/oder 2, dadurch gekennzeichnet, daß die als Substituent an eine für $R^2$ stehende Alkylgruppe gebundenen Carbonsäuregruppierungen Amide, wie das unsubstituierte Amid, Mono-und Dialkylamide mit 1 bis 3 C-Atomen pro Alkylrest, Ester, wie der Methyl- oder Ethylrest, die Nitrilgruppe oder die Carboxygruppe bedeuten.

4. Pyrrolaldehyde nach einem oder mehreren der Ansprüche 1 bis 3, dadurch gekennzeichnet, daß die für $R^3$ stehenden oder als Substituent an einen für $R^2$ stehenden Alkylrest gebundenen, gegebenenfalls substituierten aliphatischen oder aromatischen 5-bis 7-gliedrigen Heterocyclen als Heteroglieder $>O$, $>S$ oder $>NR^4$ enthalten, worin $R^4$ für Wasserstoff, Alkyl mit 1 bis 4 C-Atomen oder Alkoxycarbonyl mit 1 bis 4 C-Atomen in der Alkoxygruppe steht, wobei diese, sofern der Heterocyclus 2 Heteroglieder aufweist, gleich oder verschieden sind und von den 2 Heterogliedern eines bevorzugt $>NR^4$ ist.

5. Pyrrolaldehyde nach einem oder mehreren der Ansprüche 1 bis 4, dadurch gekennzeichnet, daß sich ein heterocyclischer Rest ableitet von Pyrrol, Pyrrolidin, Imidazol, Thiazol, Thiazolidin oder Perhydrothiazepin und daß sich daneben ein heterocyclischer Rest, der als Substituent an eine für $R^2$ stehende Alkylgruppe gebunden ist, auch von Indol oder Chinazolin ableitet.

6. Pyrrolaldehyd nach einem oder mehreren der Ansprüche 1 bis 5, dadurch gekennzeichnet, daß ein für $R^3$ stehender Phenylrest, ein für $R^3$ stehender Phenylaminorest und ein als Substituent an einer für $R^2$ oder $R^3$ stehenden Alkylgruppe gebundener Phenoxyrest im Kern bis zu drei Substituenten tragt, und zwar Chlor, Alkyl und Alkoxy mit 1 oder 2 C-Atomen, insbesondere Methyl oder Methoxy, Carboxy und Alkoxycarbonyl mit 1 oder 2 C-Atomen in der Alkoxygruppe, als gegebenenfalls zweiten Substituenten des Kerns eine der oben definierten Alkyl-oder Alkoxygruppen oder Chlor und als gegebenenfalls dritten Substituenten eine der obigen Alkyl-oder Alkoxygruppen.

7. Verfahren zur Herstellung von Pyrrolaldehyden der allgemeinen Formel I

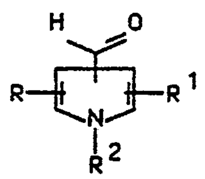

(I)

worin R, $R^1$ und $R^2$ die in den Ansprüchen 1 bis 6 angegebene Bedeutungen haben, bzw. ihrer physiologisch verträglichen Säureadditionssalze, dadurch gekennzeichnet, daß man Pyrrole der allgemeinen Formel III

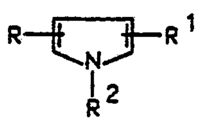

(III)

in an sich bekannter Weise formyliert und, softer $R^2$ ein durch einen Rest der allgemeinen Formel II

$$-NH-\overset{\overset{X}{\|}}{C}-R^3 \qquad (II)$$

substituierter Alkylrest ist, gewünschtenfalls der Acylrest $-CX-R^3$ in an sich bekannter Weise hydrolytisch abspaltet und/oder gewünschenfalls die Verbindung in an sich bekannter Weise in ein pharmakologisch verträgliches Säureadditionssalz überführt.

8. Verfahren zur Herstellung von Pyrrolaldehyden der allgemeinen Formel I des Anspruchs 1, in der $R^2$ ein durch eine Acylaminogruppe der allgemeinen Formel II substituierter Alkylrest mit 1 bis 3 C-Atom ist und R und $R^1$ die in den Ansprüchen 1 und 2 angegebenen Bedeutungen haben, bzw. ihrer physiologisch verträglichen Säureadditionssalze, dadurch gekennzeichnet, daß man ein Aminoalkylpyrrol der allgemeinen Formel V

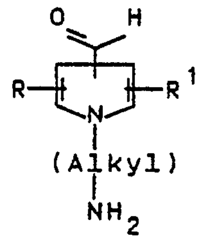

(V)

mit einem reaktiven Carbonsäurederivat, abgeleitet von einer Carbonsäure der allgemeinen Formel VI

$R^3$ - COOH (VI)

worin $R^3$ die in den Ansprüchen 1 und 2 angegebene Bedeutung hat, oder mit einem Alkalicyanat bzw. -thiocyanat oder mit einem Isocyanat bzw. Isothiocyanat der allgemeinen Formel VII

$R^5$ -NCX (VII)

worin X Sauerstoff oder Schwefel und $R^5$ Phenyl oder Alkyl, das gegebenenfalls in der in Anspruch 2 präzisierten Weise substituiert ist, bedeutet, acyliert werden und gewünschtenfalls die Verbindung in an sich bekannter Weise in ein pharmakologisch verträgliches Säueradditionssalz überführt.

9. Verwendung eines Pyrrolaldehyds der allgemeinen Formel I

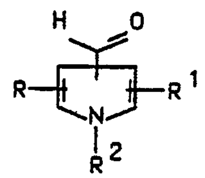

(I)

worin R und $R^1$ unabhängig voneinander Wasserstoff oder Alkyl mit 1 bis 4 C-Atomen,
$R^2$ Alkyl, das durch $-NH_2$, Acylamino der allgemeinen Formel II

$-NH-\overset{X}{\overset{\|}{C}}-R^3$ (II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und
$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Phenylaminogruppe bedeutet, oder eines pharmazeutisch akzeptablen Säureadditionssalzes, zur Herstellung eines Arzneimittels zur Bekämpfung und vorbeugung von Erkrankungen, die durch Einschränkung der Gehirnfunktion bedingt sind, oder zur Behandlung von cerebralen Alterungsvorgängen.

10. Pharmazeutisches Präparat, dadurch gekennzeichnet, daß es als Wirkstoff ein Pyrrolaldehyd der allgemeinen Formel I

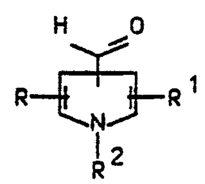

(I)

worin R und $R^1$ unabhängig voneinander Wasserstoff oder Alkyl mit 1 bis 4 C-Atomen,
$R^2$ Alkyl, das durch $-NH_2$, Acylamino der Formel II

$-NH-\overset{X}{\overset{\|}{C}}-R^3$ (II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und

$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Phenylaminogruppe bedeutet, oder ein pharmazeutisch akzeptables Säureadditionssalz davon, zusammen mit einem pharmazeutisch annehmbaren Trägerstoff und gegebenenfalls pharmazeutisch annehmbaren Zusatzstoffen und gegebenenfalls noch einen oder mehrere andere pharmakologische Wirkstoffe enthält.

Patentansprüche für den folgenden Vertragsstaat: ES

1. Verfahren zur Herstellung von Pyrrolaldehyden der allgemeinen Formel I

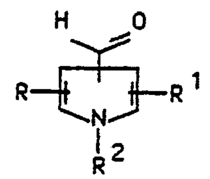

(I)

worin R und $R^1$ unabhängig voneinander Wasserstoff oder Alkyl mit 1 bis 4 C-Atomen,
$R^2$ Alkyl, das durch $-NH_2$, Acylamino der allgemeinen Formel II

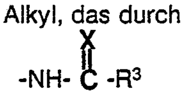 (II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und
$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Phenylaminogruppe bedeutet, sowie deren pharmazeutisch akzeptable Säureadditionssalze, wobei für die Fälle, in denen für $R^2$ stehendes Alkyl durch eine Carbonsäuregruppierung substituiert ist, für R und $R^1$ Alkyl stehen muß und wobei $R^3$ nicht für Wasserstoff stehen kann, wenn R und $R^1$ Wasserstoff bedeuten und der Pyrrolring in 3-Stellung formyliert ist, bzw. ihrer physiologisch verträglichen Säureadditionssalze, dadurch gekennzeichnet, daß man Pyrrole der allgemeinen Formel III

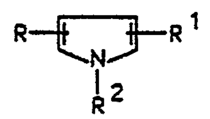

(III)

in an sich bekannter Weise formyliert und, sofern $R^2$ ein durch einen Rest der allgemeinen Formel II

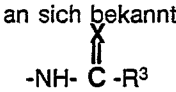 (II)

substituierter Alkylrest ist, gewünschtenfalls der Acylrest $-CX-R^3$ in an sich bekannter Weise hydrolytisch abspaltet und/oder gewünschenfalls die Verbindung in an sich bekannter Weise in ein pharmakologisch verträgliches Säureadditionssalz überführt.

2. Verfahren zur Herstellung von Pyrrolaldehyden der allgemeinen Formel I des Anspruchs 1, in der $R^2$ ein durch eine Acylaminogruppe der allgemeinen Formel II substituierter Alkylrest mit 1 bis 3 C-Atomen ist und R und $R^1$ die in den Ansprüchen 1 und 2 angegebenen Bedeutungen haben, bzw. ihrer physiologisch verträglichen Säureadditionssalze, dadurch gekennzeichnet, daß man ein Aminoalkylpyrrol der allgemeinen Formel V

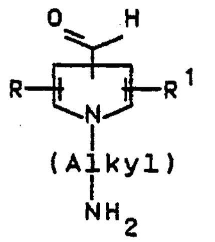

(V)

mit einem reaktiven Carbonsäurederivat, abgeleitet von einer Carbonsäure der allgemeinen Formel VI

$R^3$ - COOH (VI)

worin $R^3$ die in den Ansprüchen 1 und 2 angegebene Bedeutung hat, oder mit einem Alkalicyanat bzw. -thiocyanat oder mit Isocyanat bzw. Isothiocyanat der allgemeinen Formel VII

$R^5$ -NCX (VII)

worin X Sauerstoff oder Schwefel und $R^5$ Phenyl oder Alkyl, das gegebenenfalls in der Anspruch 2 präzisierten Weise substituiert ist, bedeutet, acyliert werden und gewünschtenfalls die Verbindung in an sich bekannter Weise in ein pharmakologisch verträgliches Säureadditionssalz überführt.

3. Verfahren gemäß Anspruch 1, dadurch gekennzeichnet, daß die als Substituent an eine für $R^2$ stehende Alkylgruppe gebundenen Carbonsäuregruppierungen Amide, wie das unsubstituierte Amid, MonoundDialkylamide mit 1 bis 3 C-Atomen pro Alkylrest, Ester, wie der Methyl-oder Ethylrest, die Nitrilgruppe oder die Carboxygruppe bedeuten.

4. Verfahren nach einem oder mehreren der Ansprüche 1 bis 3, dadurch gekennzeichnet, daß die für $R^3$ stehenden oder als Substituent an einen für $R^2$ stehenden Alkylrest gebundenen, gegebenenfalls substituierten aliphatischen oder aromatischen 5-bis 7-gliedrigen Heterocyclen als Heteroglieder ⋝O, ⋝S oder ⋝$NR^4$ enthalten, worin $R^4$ für Wasserstoff, Alkyl mit 1 bis 4 C-Atomen oder Alkoxycarbonyl mit 1 bis 4 C-Atomen in der Alkoxygruppe steht, wobei diese, sofern der Heterocyclus 2 Heteroglieder aufweist, gleich oder verschieden sind und von den 2 Heterogliedern eines bevorzugt ⋝$NR^4$ ist.

5. Verfahren nach einem oder mehreren der Ansprüche 1 bis 4, dadurch gekennzeichnet, daß sich ein heterocyclischer Rest ableitet von Pyrrol, Pyrrolidin, Imidazol, Thiazol, Thiazolidin oder Perhydrothiazepin und daß sich daneben ein heterocyclischer Rest, der als Substituent an eine für $R^2$ stehende Alkylgruppe gebunden ist, auch von Indol oder Chinazolin ableitet.

6. Verfahren nach einem oder mehreren der Ansprüche 1 bis 5, dadurch gekennzeichnet, daß ein für $R^3$ stehender Phenylrest, ein für $R^3$ stehender Phenylaminorest und ein als Substituent an einer für $R^2$ oder $R^3$ stehenden Alkylgruppe gebundener Phenoxyrest im Kern bis zu drei Substituenten tragt, und zwar Chlor, Alkyl und Alkoxy mit 1 oder 2 C-Atomen, insbesondere Methyl oder Methoxy, Carboxy und Alkoxycarbonyl mit 1 oder 2 C-Atomen in der Alkoxygruppe, als gegebenenfalls zweiten Substituenten des Kerns eine der oben definierten Alkyl-oder Alkoxygruppen oder Chlor und als gegebenenfalls dritten Substituenten eine der obigen Alkyl-oder Alkoxygruppen.

7. Verwendung eines Pyrrolaldehyds der allgemeinen Formel I

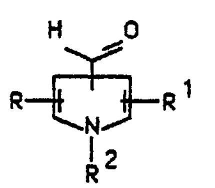

(I)

worin R und $R^1$ unabhängig voneinander Wasserstoff oder Alkyl mit 1 bis 4 C-Atomen,

$R^2$ Alkyl, das durch $-NH_2$, Acylamino der allgemeinen Formel II

$$-NH-\overset{\overset{X}{\|}}{C}-R^3 \quad (II)$$

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,

X für ein Sauerstoff-oder Schwefelatom steht und

$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Amino gruppe oder eine gegebenenfalls

substituierte Phenylaminogruppe bedeutet, oder eine pharmazeutisch akzeptables Säureadditionssalz zur Herstellung eines Arzneimittels zur Bekämpfung und Vorbeugung von Erkrankungen, die durch Einschränkung der Gehirnfunktion bedingt sind, oder zur Behandlung von cerebralen Alterungsvorgängen.

8. Verfahren zur Herstellung eines pharmazeutischen Präparates, dadurch gekennzeichnet, daß eine oder mehrere Verbindung der allgemeinen Formel I

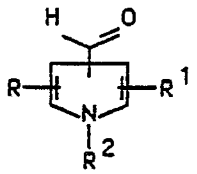

(I)

Worin R und $R^1$ unabhängig voneinander Wasserstoff oder Alkyl mit 1 bis 4 C-Atomen,
$R^2$ Alkyl, das durch $-NH_2$, Acylamino der allgemeinen Formel II

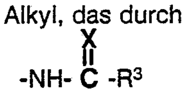

(II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und
$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Phenylaminogruppe bedeutet, oder ein pharmazeutisch akzeptables Säureadditionssalz davon, durch Mischen mit geeigneten pharmazeutisch zulässigen Träger-und Zusatzstoffen und gegebenenfalls noch einer oder mehreren anderen pharmazeutisch wirksamen Substanzen in eine zur Verarbreichung geeignete Form gebracht werden.

Patentansprüche für den folgenden Vertragsstaat: GR

1. Pyrrolaldehyde der allgemeinen Formel I

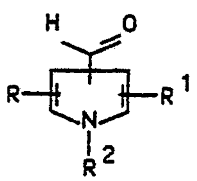

(I)

worin R und $R^1$ unabhängig voneinander Wasserstoff oder Alkyl mit 1 bis 4 C-Atomen,
$R^2$ Alkyl, das durch $-NH_2$, Acylamino der allgemeinen Formel II

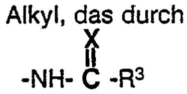

(II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und
$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Phenylaminogruppe bedeutet, sowie deren pharmazeutisch akzeptable Säureadditionssalze, wobei für die Fälle, in denen für $R^2$ stehendes Alkyl durch eine Carbonsäuregruppierung substituiert ist, für R und $R^1$ Alkyl stehen muß und wobei $R^3$ nicht für Wasserstoff stehen kann, wenn R und $R^1$ Wasserstoff bedeuten und der Pyrrolring in 3-Stellung formyliert ist.

2. Pyrrolaldehyde gemäß Anspruch 1 der allgemeinen Formel I

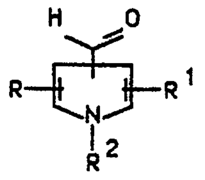

(I)

worin R und $R^1$ unabhängig voneinander (Wasserstoff oder Alkyl mit 1 bis 4 C-Atomen, $R^2$ Alkyl mit 1 bis 3 C-Atomen, das durch $-NH_2$, Acylamino der allgemeinen Formel II

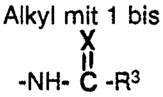

(II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeutet,

X für ein Sauerstoff-oder Schwefelatom steht und

$R^3$ Wasserstoff, Alkyl oder Alkylamino mit 1 bis 5, vorzugsweise 1 oder 2, C-Atomen, das gegebenenfalls durch $-NH_2$, Monoalkylamino mit 1 bis 4, vorzugsweise 1 oder 2, C-Atomen, Dialkylamino mit insgesamt 2 bis 6, vorzugsweise 2 bis 4, C-Atomen, einen N-Pyrrolidinyl-, N-Piperidinyl-, N-Morpholinyl-, N-Thiomorpholinyl-oder durch einen in 4-Stellung gegebenenfalls durch Alkyl mit 1 bis 4 C-Atomen, Phenyl, Toluyl, Chlorphenyl oder Methoxy-oder Ethoxyphenyl substituierten Piperazin-1-yl-Rest, durch Alkoxy mit 1 bis 4, vorzugsweise 1 oder 2, C-Atomen oder durch gegebenenfalls substituiertes Phenoxy substituiert ist; Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Alkyl-oder Phenylaminogruppe bedeutet, wobei für die Fälle, in denen für $R^2$ stehendes Alkyl durch eine Carbonsäuregruppierung substituiert ist, für R und $R^1$ Alkyl stehen muß und wobei $R^3$ nicht für Wasserstoff stehen kann, wenn R und $R^1$ Wasserstoff bedeuten und der Pyrrolring in 3-Stellung formyliert ist.

3. Pyrrolaldehyde gemäß den Ansprüchen 1 und/oder 2, dadurch gekennzeichnet, daß die als Substituent an eine für $R^2$ stehende Alkylgruppe gebundenen Carbonsäuregruppierungen Amide, wie das unsubstituierte Amid, Mono-und Dialkylamide mit 1 bis 3 C-Atomen pro Alkylrest, Ester, wie der Methyl-oder Ethylrest, die Nitrilgruppe oder die Carboxygruppe bedeuten.

4. Pyrrolaldehyde nach einem oder mehreren der Ansprüche 1 bis 3, dadurch gekennzeichnet, daß die für $R^3$ stehenden oder als Substituent an einen für $R^2$ stehenden Alkylrest gebundenen, gegebenenfalls substituierten aliphatischen oder aromatischen 5-bis 7-gliedrigen Heterocyclen als Heteroglieder $\geq$O, $\geq$S oder $\geq NR^4$ enthalten, worin $R^4$ für Wasserstoff, Alkyl mit 1 bis 4 C-Atomen oder Alkoxycarbonyl mit 1 bis 4 C-Atomen in der Alkoxygruppe steht, wobei diese, sofern der Heterocyclus 2 Heteroglieder aufweist, gleich oder verschieden sind und von den 2 Heterogliedern eines bevorzugt $\geq NR^4$ ist.

5. Pyrrolaldehyde nach einem oder mehreren der Ansprüche 1 bis 4, dadurch gekennzeichnet, daß sich ein heterocyclischer Rest ableitet von Pyrrol, Pyrrolidin, Imidazol, Thiazol, Thiazolidin oder Perhydrothiazepin und daß sich daneben ein heterocyclischer Rest, der als Substituent an eine für $R^2$ stehende Alkylgruppe gebunden ist, auch von Indol oder Chinazolin ableitet.

6. Pyrrolaldehyd nach einem oder mehreren der Ansprüche 1 bis 5, dadurch gekennzeichnet, daß ein für $R^3$ stehender Phenylrest, ein für $R^3$ stehender Phenylaminorest und ein als Substituent an einer für $R^2$ oder $R^3$ stehenden Alkylgruppe gebundener Phenoxyrest im Kern bis zu drei Substituenten tragt, und zwar Chlor, Alkyl und Alkoxy mit 1 oder 2 C-Atomen, insbesondere Methyl oder Methoxy, Carboxy und Alkoxycarbonyl mit 1 oder 2 C-Atomen in der Alkoxygruppe, als gegebenenfalls zweiten Substituenten des Kerns eine der oben definierten Alkyl-oder Alkoxygruppen oder Chlor und als gegebenenfalls dritten Substituenten eine der obigen Alkyl-oder Alkoxygruppen.

7. Verfahren zur Herstellung von Pyrrolaldehyden der allgemeinen Formel I

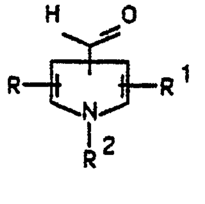

(I)

worin R, $R^1$ und $R^2$ die in den Ansprüchen 1 bis 6 angegebene Bedeutungen haben, bzw. ihrer physiologisch verträglichen Säureadditionssalze, dadurch gekennzeichnet, daß man Pyrrole der allgemeinen Formel III

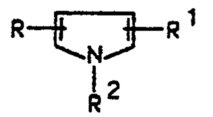

(III)

in an sich bekannter Weise formyliert und, softer $R^2$ ein durch einen Rest der allgemeinen Formel II

$-NH-\overset{X}{\overset{\|}{C}}-R^3$ (II)

substituierter Alkylrest ist, gewünschtenfalls der Acylrest $-CX-R^3$ in an sich bekannter Weise hydrolytisch abspaltet und/oder gewünschenfalls die Verbindung in an sich bekannter Weise in ein pharmakologisch verträgliches Säureadditionssalz überführt.

8. Verfahren zur Herstellung von Pyrrolaldehyden der allgemeinen Formel I des Anspruchs 1, in der $R^2$ ein durch eine Acylaminogruppe der allgemeinen Formel II substituierter Alkylrest mit 1 bis 3 C-Atom ist und R und $R^1$ die in den Ansprüchen 1 und 2 angegebenen Bedeutungen haben, bzw. ihrer physiologisch verträglichen Säureadditionssalze, dadurch gekennzeichnet, daß man ein Aminoalkylpyrrol der allgemeinen Formel V

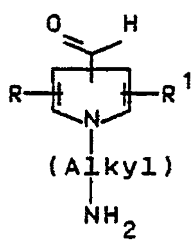

(V)

mit einem reaktiven Carbonsäurederivat, abgeleitet von einer Carbonsäure der allgemeinen Formel VI

$R^3$ - COOH (VI)

worin $R^3$ die in den Ansprüchen 1 und 2 angegebene Bedeutung hat, oder mit einem Alkalicyanat bzw. -thiocyanat oder mit einem Isocyanat bzw. Isothiocyanat der allgemeinen Formel VII

$R^5$ -NCX (VII)

worin X Sauerstoff oder Schwefel und $R^5$ Phenyl oder Alkyl, das gegebenenfalls in der in Anspruch 2 präzisierten Weise substituiert ist, bedeutet, acyliert werden und gewünschtenfalls die Verbindung in an sich bekannter Weise in ein pharmakologisch verträgliches Säueradditionssalz überführt.

9. Verwendung eines Pyrrolaldehyds der allgemeinen Formel I

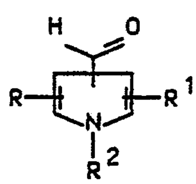

(I)

worin R und $R^1$ unabhängig voneinander Wasserstoff oder Alkyl mit 1 bis 4 C-Atomen,
$R^2$ Alkyl, das durch $-NH_2$, Acylamino der allgemeinen Formel II

$-NH-\overset{X}{\overset{\|}{C}}-R^3$ (II),

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und
$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls

substituierte Phenylaminogruppe bedeutet, sowie deren pharmazeutisch akzeptablen Säureadditionssalzes, zur Herstellung eines Arzneimittels zur Bekämpfung und Vorbeugung von Erkrankungen, die durch Einschränkung der Gehirnfunktion bedingt sind, oder zur Behandlung von cerebralen Alterungsvorgängen.

10. Verfahren zur Herstellung eines pharmazeutischen Präparats, dadurch gekennzeichnet, daß eine oder mehrere Verbindungen der allgemeinen Formel I

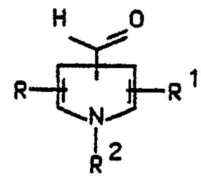

(I)

worin R und $R^1$ unabhängig voneinander Wasserstoff oder Alkyl mit 1 bis 4 C-Atomen,
$R^2$ Alkyl, das durch $-NH_2$, Acylamino der allgemeinen Formel II

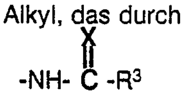

(II)

eine Carbonsäuregruppierung, einen gegebenenfalls substituierten aliphatischen oder aromatischen 5-bis 7-gliedrigen Heterocyclus mit 1 oder 2 Heterogliedern oder eine gegebenenfalls substituierte Phenoxygruppe substituiert ist, bedeuten,
X für ein Sauerstoff-oder Schwefelatom steht und
$R^3$ Wasserstoff, gegebenenfalls substituiertes Alkyl oder Alkylamino, Cycloalkyl mit 5 bis 7 C-Atomen, gegebenenfalls substituiertes Phenyl, einen gegebenenfalls substituierten aliphatischen oder aromatischen 5-bis 7-gliedrigen Heterocyclus mit 1 oder 2 Heteroatomen, eine Aminogruppe oder eine gegebenenfalls substituierte Phenylaminogruppe bedeutet, oder ein pharmazeutisch akzeptables Säureadditionssalz davon, durch Mischen mit geeigneten pharmazeutisch zulässigen Träger-und Zusatstoffen und gegebenenfalls noch einer mehreren anderen pharmazeutisch wirksamen Substanzen in eine zur Verabreichung geeignete Form gebracht werden.